(19) Europäisches Patentamt
European Patent Office
Office européen des brevets

(11) Veröffentlichungsnummer: **0 106 093**
**B1**

(12) # EUROPÄISCHE PATENTSCHRIFT

(45) Veröffentlichungstag der Patentschrift: **20.11.86**

(51) Int. Cl.⁴: **C 07 C 161/00**, C 07 D 295/22, A 01 N 41/02, A 01 N 43/34, A 01 N 43/84

(21) Anmeldenummer: **83108647.5**

(22) Anmeldetag: **02.09.83**

(54) **Jodpropargylsulfamide.**

(30) Priorität: **14.09.82 DE 3234037**

(43) Veröffentlichungstag der Anmeldung:
**25.04.84 Patentblatt 84/17**

(45) Bekanntmachung des Hinweises auf die Patenterteilung:
**20.11.86 Patentblatt 86/47**

(84) Benannte Vertragsstaaten:
**BE CH DE FR GB IT LI NL SE**

(56) Entgegenhaltungen:
**DE - A - 1 964 441**

(73) Patentinhaber: **BAYER AG, Konzernverwaltung RP Patentabteilung, D-5090 Leverkusen 1 Bayerwerk (DE)**

(72) Erfinder: **Schmitt, Hans-Georg, Dr., Gustav-Freytag-Strasse 2, D-5090 Leverkusen 1 (DE)**
Erfinder: **Paulus, Wilfried, Dr., Deswatinesstrasse 90, D-4150 Krefeld 1 (DE)**
Erfinder: **Genth, Hermann, Dr., Am Heckerhof 60, D-4150 Krefeld 1 (DE)**
Erfinder: **Brandes, Wilhelm, Dr., Eichendorffstrasse 3, D-5653 Leichlingen 1 (DE)**
Erfinder: **Reinecke, Paul, Dr., Lessingstrasse 11, D-5090 Leverkusen 3 (DE)**
Erfinder: **Scheinpflug, Hans, Dr., Am Thelenhof 15, D-5090 Leverkusen 1 (DE)**



Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Die vorliegende Erfindung betrifft neue Jodpropargylsulfamide, ein Verfahren zu ihrer Herstellung und ihre Verwendung als Schädlingsbekämpfungsmittel.

Es ist bereits bekannt geworden, dass eine Reihe von organischen Verbindungen, wie z.B. Zinkethylen-1,2-bis-(dithiocarbamat) oder N-Trichlormethylthio-tetrahydrophthalimid, fungizide und bakterizide Eigenschaften aufweisen (vgl. z.B. R. Wegler, «Chemie der Pflanzenschutz- und Schädlingsbekämpfungsmittel», Springer Verlag Chemie, Band 2). Die Wirkung ist jedoch insbesondere bei niedrigen Aufwandmengen und -konzentrationen nicht immer voll befriedigend.

Ferner sind N-Jodpropargylsulfonsäureamide mit einer mikrobiziden Wirksamkeit beschrieben (vgl. Japanische Anmeldung 74 100 037). Weiterhin sind Dichlorfluormethansulfenylsulfamide bekannt, die fungizide Wirksamkeit besitzen und im Pflanzenschutz Anwendung finden und ausserdem können sie in mikrobiziden Mitteln zum Schutz technischer Materialien Verwendung finden (vgl. «Angewandte Chemie», 76, 807 (1964) bzw. Niederländische Patentschrift 6 504 561).

Es wurden neue Jodpropargylsulfamide der allgemeinen Formel (I) gefunden

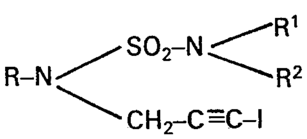

(I)

in welcher

R für Wasserstoff, für geradkettiges oder verzweigtes Alkyl mit 1 bis 18 Kohlenstoffatomen, für gegebenenfalls ein- bis fünffach durch Alkyl mit 1 bis 6 Kohlenstoffatomen substituiertes Cycloalkyl mit 3 bis 8 Ringgliedern, für gegebenenfalls ein- bis fünffach, gleich oder verschieden durch Alkyl mit 1 bis 4 Kohlenstoffatomen und/oder Halogen am Phenylring substituiertes Phenylalkyl mit 1 bis 4 Kohlenstoffatomen im Alkylteil, für gegebenenfalls ein- bis vierfach, gleich oder verschieden durch Alkyl mit 1 bis 4 Kohlenstoffatomen und/oder Halogen substituiertes Heterocyclylalkyl mit 1 bis 4 Kohlenstoffatomen im Alkylteil und mit 5 bis 8 Ringgliedern im Heterocyclylteil, wobei der Heterocyclylteil 1 bis 3 Heteroatome, wie Stickstoff, Sauerstoff und/oder Schwefel, enthalten kann, ferner für Phenyl, das ein- bis fünffach, gleich oder verschieden durch Halogen, Cyano, Nitro, Alkyl mit 1 bis 5 Kohlenstoffatomen, Alkoxy mit 1 bis 5 Kohlenstoffatomen, Alkylthio mit 1 bis 5 Kohlenstoffatomen, Alkoxycarbonyl mit 1 bis 5 Kohlenstoffatomen im Alkoxyteil, Halogenalkyl oder Halogenalkoxy bzw. Halogenalkylthio oder Halogenalkylsulfonyl mit 1 bis 5 Kohlenstoffatomen und 1 bis 5 Halogenatomen je Halogenalkylrest substituiert sein kann,

$R^1$ und $R^2$ für gleiches oder verschiedenes Alkyl mit je 1 bis 5 Kohlenstoffatomen oder

$R^1$ und $R^2$ für eine Alkylenbrücke mit 2 bis 8 Kohlenstoffatomen, die mit dem Stickstoff, an dem sie stehen, einen Ring bilden, der durch weitere Heteroatome wie Sauerstoff, Schwefel, die Gruppen SO bzw. $SO_2$ oder durch N-Alkyl unterbrochen sein kann. Die Ringe können gegebenenfalls ein- bis fünffach durch Alkyl mit 1 bis 6 Kohlenstoffatomen substituiert sein.

Weiterhin wurde gefunden, dass man die neuen Jodpropargylsulfamide der Formel (I)

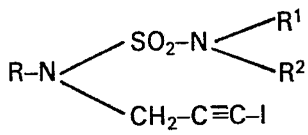

(I)

in welcher

R für Wasserstoff, für geradkettiges oder verzweigtes Alkyl mit 1 bis 18 Kohlenstoffatomen, für gegebenenfalls ein- bis fünffach durch Alkyl mit 1 bis 6 Kohlenstoffatomen substituiertes Cycloalkyl mit 3 bis 8 Ringgliedern, für gegebenenfalls ein- bis fünffach, gleich oder verschieden durch Alkyl mit 1 bis 4 Kohlenstoffatomen und/oder Halogen am Phenylring substituiertes Phenylalkyl mit 1 bis 4 Kohlenstoffatomen im Alkylteil, für gegebenenfalls ein- bis vierfach, gleich oder verschieden durch Alkyl mit 1 bis 4 Kohlenstoffatomen und/oder Halogen substituiertes Heterocyclylalkyl mit 1 bis 4 Kohlenstoffatomen im Alkylteil und mit 5 bis 8 Ringgliedern im Heterocyclylteil, wobei der Heterocyclylteil 1 bis 3 Heteroatome, wie Stickstoff, Sauerstoff und/oder Schwefel, enthalten kann, ferner für Phenyl, das ein- bis fünffach, gleich oder verschieden durch Halogen, Cyano, Nitro, Alkyl mit 1 bis 5 Kohlenstoffatomen, Alkoxy mit 1 bis 5 Kohlenstoffatomen, Alkylthio mit 1 bis 5 Kohlenstoffatomen, Alkoxycarbonyl mit 1 bis 5 Kohlenstoffatomen im Alkoxyteil, Halogenalkyl oder Halogenalkoxy bzw. Halogenalkylthio oder Halogenalkylsulfonyl mit 1 bis 5 Kohlenstoffatomen und 1 bis 5 Halogenatomen je Halogenalkylrest substituiert sein kann,

$R^1$ und $R^2$ für gleiches oder verschiedenes Alkyl mit je 1 bis 5 Kohlenstoffatomen oder

$R^1$ und $R^2$ für eine Alkylenbrücke mit 2 bis 8 Kohlenstoffatomen, die mit dem Stickstoff, an dem sie stehen, einen Ring bilden, der durch weitere Heteroatome wie Sauerstoff, Schwefel, die Gruppen SO bzw. $SO_2$ oder durch N-Alkyl unterbrochen sein kann. Die Ringe können gegebenenfalls ein- bis fünffach durch Alkyl mit 1 bis 6 Kohlenstoffatomen substituiert sein,

erhält, wenn man Propargylsulfamide der Formel (II)

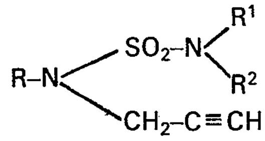

(II)

in welcher

R, $R^1$ und $R^2$ die oben angegebene Bedeutung

haben, in Gegenwart einer Base gegebenenfalls in Gegenwart eines Lösungs- oder Verdünnungsmittels mit Jod umsetzt.

Die neuen Jodpropargylsulfamide der Formel (I) weisen mikrobizide Eigenschaften auf. Dabei zeigen die erfindungsgemässen Verbindungen der Formel (I) überraschenderweise eine bessere mikrobizide Wirksamkeit als die nach dem Stand der Technik bekannten fungiziden und bakteriziden Stoffe, wie u.a. das Zinkethylen-1,2-bis-(dithiocarbamat) und das N-Trichlormethylthio-tetrahydrophthalimid. Die erfindungsgemässen Stoffe stellen somit eine Bereicherung der Technik dar.

Die erfindungsgemässen Jodpropargylsulfamide sind durch die Formel (I) allgemein definiert.

Besonders bevorzugt sind die Verbindungen der Formel (I), in denen

R für Wasserstoff, für geradkettiges oder verzweigtes Alkyl mit 1 bis 12 Kohlenstoffatomen, für gegebenenfalls ein- bis dreifach durch Alkyl mit 1 bis 4 Kohlenstoffatomen substituiertes Cycloalkyl mit 3 bis 6 Ringgliedern, für gegebenenfalls ein- bis fünffach, gleich oder verschieden durch Alkyl mit 1 bis 3 Kohlenstoffatomen und/oder Fluor, Chlor, Brom oder Jod am Phenylring substituiertes Phenylalkyl mit 1 oder 2 Kohlenstoffatomen im Alkylteil, für gegebenenfalls ein- bis dreifach, gleich oder verschieden durch Methyl, Ethyl, Fluor, Chlor oder Brom substituiertes Heterocyclylalkyl mit 1 oder 2 Kohlenstoffatomen im Alkylteil und mit 5 oder 6 Ringgliedern im Heterocyclylteil, wobei der Heterocyclylteil 1 oder 2 gleiche oder verschiedene Heteroatome, wie Stickstoff oder Sauerstoff, enthalten kann, ferner für Phenyl, das ein- bis dreifach, gleich oder verschieden durch Fluor, Chlor, Brom, Cyano, Nitro, Alkyl mit 1 bis 3 Kohlenstoffatomen, wie Methyl, Ethyl, n-Propyl oder iso-Propyl, Methoxy, Ethoxy, Trifluormethyl, Trifluormethoxy, Trifluormethylthio, Difluorchlormethyl, Difluorchlormethoxy, Difluorchlormethylthio, Trifluormethylsulfonyl, Methoxycarbonyl oder Ethoxycarbonyl substituiert sein kann, steht und

$R^1$ und $R^2$ gleich oder verschieden sind und für Alkyl mit 1 bis 3 Kohlenstoffatomen stehen oder

$R^1$ und $R^2$ für eine Alkylenbrücke mit 2 bis 5 Kohlenstoffatomen stehen, die mit dem Stickstoffatom, an dem sie stehen, einen Ring bilden, der durch weitere Heteroatome, wie Sauerstoff, Schwefel oder N-Alkyl mit 1 bis 3 Kohlenstoffatomen unterbrochen sein kann.

Der Reaktionsablauf des erfindungsgemässen Verfahrens zur Herstellung der neuen Verbindungen kann durch das folgende Formelschema wiedergegeben werden, wenn man als Ausgangsstoffe N-(3-Fluorphenyl)-N-propargyl-N'-ethyl-N'-n-propyl-sulfamid und Jod in Gegenwart einer Base verwendet:

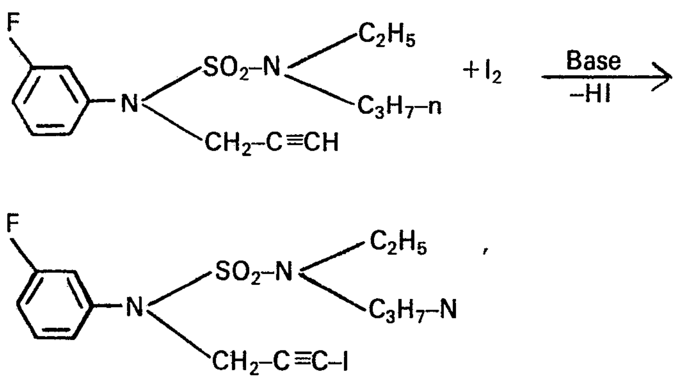


Die bei der Durchführung des erfindungsgemässen Verfahrens als Ausgangsstoffe zu verwendenden Propargylsulfamide sind durch die Formel (II) allgemein definiert. Einige dieser Verbindungen sind bekannt (vgl. Niederländische Patentanmeldung 6 500 481). Man kann auch die neuen Ausgangsverbindungen nach bekannten Verfahren herstellen, z.B. wenn man

a) ein Sulfamid der Formel (III)

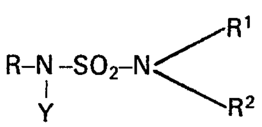 (III)

in welcher

Y für Wasserstoff oder für ein Alkaliäquivalent wie Lithium, Kalium oder Natrium, steht und

R, $R^1$ und $R^2$ die oben angegebene Bedeutung haben, mit einem Propargylhalogenid der Formel (IV)

$$Hal{-}CH_2{-}C{\equiv}CH \qquad (IV)$$

in welcher

Hal für Halogen, wie Chlor, Brom oder Jod steht, gegebenenfalls in Gegenwart einer Base umsetzt oder

b) ein Propargylamin der Formel (V)

$$R{-}NH{-}CH_2{-}C{\equiv}CH \qquad (V)$$

in welcher

R die angegebene Bedeutung hat, mit einem Sulfamoylchlorid der Formel (VI)

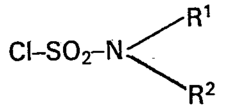

(VI)

in welcher

$R^1$ und $R^2$ die oben angegebene Bedeutung haben, umsetzt.

Das ausserdem als Ausgangsstoff zu verwendende Jod ist eine käuflich zu erwerbende Substanz.

Das erfindungsgemässe Verfahren zur Herstellung der neuen Verbindungen der Formel (I) wird in Gegenwart einer Base durchgeführt. Man kann alle üblicherweise verwendbaren Basen verwenden. Hierzu gehören z.B. Alkalihydroxide, wie Natriumhydroxid oder Kaliumhydroxid, weiterhin tertiäre Amine.

Als Lösungs- oder Verdünnungsmittel kommen für das erfindungsgemässe Verfahren vorzugsweise alle inerten Lösungsmittel in Frage. Hierzu gehören Ether, wie Dioxan, Tetrahydrofuran oder Glykoldimethylether. Weiterhin können Alkohole, wie Methanol oder Ethanol, Verwendung finden, die Umsetzung kann auch in Wasser oder bevorzugt in Gemischen aus Lösungsmittel und Wasser durchgeführt werden.

Die Reaktionstemperatur kann bei der Durchführung des erfindungsgemässen Verfahrens innerhalb eines grösseren Bereiches variiert werden. Im allgemeinen arbeitet man zwischen −10 °C und 40 °C, vorzugsweise zwischen 0 °C und 20 °C.

Das erfindungsgemässe Verfahren wird im allgemeinen bei Normaldruck durchgeführt.

Zur Durchführung des erfindungsgemässen Verfahrens setzt man auf 1 Mol Propargylsulfamid der Formel (II) gewöhnlich zwischen 1 und 1,5 Mol, bevorzugt zwischen 1,0 und 1,3 Mol Jod, ein.

Zur Durchführung des erfindungsgemässen Verfahrens setzt man auf 1 Mol Propargylsulfamid der Formel (II) gewöhnlich zwischen 1 und 10 Mol, vorzugsweise zwischen 1 und 5 Mol, Base ein.

Die Umsetzung wird vorzugsweise so durchgeführt, dass ein Propargylsulfamid der Formel (II) in einem der oben angegebenen Lösungsmittel oder deren Gemisch mit Wasser vorgelegt wird und eine der oben angegebenen Basen unter Einhaltung der angegebenen Temperatur zugetropft wird. Anschliessend wird das Jod zugegeben und das Reaktionsgemisch einige Zeit bei der erforderlichen Temperatur nachgerührt. Die Aufarbeitung der Reaktionsmischung und die Isolierung der erfindungsgemässen Reaktionsprodukte der Formel (I) erfolgt in allgemein üblicher Art und Weise.

Die erfindungsgemässen Verbindungen sind zum Teil Feststoffe, zum Teil schwer kristallisierende Öle.

Zur Herstellung der Ausgangsprodukte der Formel (II) werden wie oben beschrieben Sulfamide (vgl. Niederländisches Patent 6 414 819) mit Propargylhalogeniden in Gegenwart einer Base umgesetzt. Als Basen eignen sich vor allem tertiäre Amine, wie Triethylamin und Dimethylanilin, oder Carbonate, z.B. Kalium- oder Natriumcarbonat und als Lösungsmittel kommen in Frage: Alkohole, z.B. Ethanol, Propanol und Butanol, oder Ether, z.B. Dioxan und Tetrahydrofuran, oder Kohlenwasserstoffe z.B. Toluol, Chlorkohlenstoffwasserstoffe, wie Chloroform oder Amide, wie Dimethylformamid.

In einer bevorzugten Ausführungsform wird die Umsetzung der Sulfamide der Formel (III) mit einem Propargylhalogenid in Gegenwart von Natriumhydroxid oder Kaliumhydroxid als Base und eines Phasen-Transfer-Katalysators wie Tetrabutylammoniumbromid, Triethylbenzylammoniumchlorid oder Methyltrioctylammoniumchlorid durchgeführt. Als Verdünnungsmittel dient Wasser.

In einer besonders bevorzugten Ausführungsform werden Alkalisalze der Sulfamide der allgemeinen Formel (III) mit einem Propargylhalogenid umgesetzt.

Bevorzugte Alkalisalze sind das Natrium- und das Kaliumsalz. Die Salze lassen sich durch Hinzufügen einer entsprechenden Base z.B. in wässriger, alkoholischer oder etherischer Verdünnung aus den Grundverbindungen herstellen.

Bevorzugte Propargylhalogenide sind 3-Chlor-1-propin und 3-Brom-1-propin.

Als Verdünnungsmittel finden Alkohole wie Methanol und Ethanol, Ether, wie Dioxan und Glykoldimethylether oder Amide, wie Dimethylformamid, Verwendung.

Die Reaktionstemperaturen können über einen grösseren Bereich variiert werden, im allgemeinen arbeitet man zwischen 20 °C und 100 °C, vorzugsweise bei 40 °C–80 °C.

Bei der Durchführung des Verfahrens zur Herstellung der Ausgangsverbindungen werden die Komponenten im allgemeinen in äquimolaren Mengen eingesetzt, jedoch ist es bisweilen von Vorteil, zur Vervollständigung der Reaktion das Propargylhalogenid in einem Überschuss von bis zu 50% einzusetzen.

Die Aufarbeitung kann beispielsweise wie folgt durchgeführt werden:

Nach Beendigung der Reaktion wird das Verdünnungsmittel durch Destillation entfernt, der Rückstand in einem Halogenkohlenwasserstoff wie Chloroform oder Methylenchlorid aufgenommen, die organische Phase mit Wasser gewaschen und anschliessend das Lösungsmittel eingeengt.

Die Propargylsulfamide sind zum Teil Feststoffe, zum Teil Öle, die gegebenenfalls durch Destillation bei niedrigem Druck und Temperaturen unter 100 °C gereinigt werden können.

Die zur Herstellung der Ausgangsprodukte der Formel (II) benötigten Propargylamine der Formel (V) sind allgemein bekannt oder lassen sich nach literaturbekannten Verfahren herstellen (vgl. z.B. Liebigs Annalen Chem. 576, 35, (1952) ). Auch bei dem Verfahren b) zur Herstellung der Ausgangsverbindungen der Formel (II) arbeitet man vorteilhaft in Gegenwart einer Base, wobei

vor allem tertiäre Amine, wie Triethylamin oder Dimethylanilin in Frage kommen.

Als Verdünnungsmittel können inerte Lösungsmittel eingesetzt werden. Hierzu gehören Ether wie Tetrahydrofuran und Dioxan, Kohlenwasserstoffe wie Toluol oder Amide wie Dimethylformamid und Dimethylacetamid.

Im allgemeinen wird die Reaktion bei 20–100 °C durchgeführt, vorzugsweise bei 40–80 °C.

Die Reaktionskomponenten werden üblicherweise im molaren Verhältnis eingesetzt.

Aufgrund der starken mikrobiziden Wirkung können die erfindungsgemässen Wirkstoffe zur Bekämpfung von unerwünschten Mikroorganismen praktisch eingesetzt werden. Die Wirkstoffe sind für den Gebrauch als Pflanzenschutzmittel und als fungizide Mittel zum Schutz technischer Materialien geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Bakterizide Mittel werden im Pflanzenschutz zur Bekämpfung von Pseudomonadaceae, Rhizobiaceae, Enterobacteriaceae, Corynebacteriaceae und Streptomycetaceae eingesetzt.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemässen Wirkstoffe der Formel (I) mit besonders gutem Erfolg u.a. zur Bekämpfung von Reiskrankheiten, wie z.B. Pyricularia oryzae, eingesetzt werden, ausserdem zur Bekämpfung des Apfelschorferregers, wie z.B. Venturia inaequalis, weiterhin gegen Phytophthora infestans und Puccinia recondita.

Bei entsprechender Anwendung und Konzentration zeigen die Verbindungen auch eine bakterizide Wirksamkeit.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweisshydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemässen Wirkstoffe können in den Formulierungen oder in den verschiedenen Anwendungsformen beim Gebrauch im Pflanzenschutz in Mischung mit anderen bekannten Wirkstoffen vorliegen, wie Fungiziden, Bakteriziden, Insektiziden, Akariziden, Nematiziden, Herbiziden, Schutzstoffen gegen Vogelfrass, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie

gebrauchsfertige Lösungen, Emulsionen, Suspensionen, Pulver, Pasten und Granulate angewendet werden. Die Anwendung geschieht in üblicher Weise, z.B. durch Giessen, Tauchen, Spritzen, Sprühen, Vernebeln, Verdampfen, Injizieren, Verschlämmen, Verstreichen, Stäuben, Streuen, Trockenbeizen, Feuchtbeizen, Nassbeizen, Schlämmbeizen oder Inkrustieren.

Bei der Behandlung von Pflanzenteilen können die Wirkstoffkonzentrationen in den Anwendungsformen in einem grösseren Bereich variiert werden. Sie liegen im allgemeinen zwischen 1 und 0,0001 Gew.-%, vorzugsweise zwischen 0,5 und 0,001%.

Bei der Saatgutbehandlung werden im allgemeinen Wirkstoffmengen von 0,001 bis 50 g je Kilogramm Saatgut vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02%, am Wirkungsort erforderlich.

Die erfindungsgemässen Jodpropargylsulfamide der Formel (I) sind auch als mikrobizide Mittel zum Schutz technischer Materialien geeignet.

Die Einarbeitung der erfindungsgemässen Wirkstoffe in technische Materialien, die Angriffen durch Pilze und Bakterien ausgesetzt sind, inhibiert das Wachstum der Pilze und Bakterien; somit bleibt der ursprüngliche Wert der Materialien erhalten.

Technische Materialien im Rahmen der vorliegenden Erfindung sind Produkte, die selbst nicht in der Natur vorkommen, sondern aus natürlichen oder synthetischen Ausgangsmaterialien gefertigt werden. Die zu schützenden Produkte im Rahmen der vorliegenden Erfindung sind technische Materialien, die durch Mikroorganismen zersetzt werden können.

Technische Materialien, die durch die erfindungsgemässen Substanzen vor einer mikrobiellen Veränderung und Zerstörung geschützt werden sollen, sind beispielsweise Klebstoffe, Leime, Papiere und Kartone, Textilien, Leder, Holz, Anstrichmittel, Putze, Kühlschmiermittel und Kunststoffartikel, die von Mikroorganismen befallen und zersetzt werden können. Im Rahmen der zu schützenden Materialien seien auch Teile von Produktionsanlagen, wie beispielsweise Kühlwasser- und Kühlschmiermittelkreisläufe, genannt, deren Funktionstüchtigkeit durch Mikroorganismen beeinträchtigt werden kann. Vorzugsweise können die erfindungsgemässen Wirkstoffe zum Schutz von Klebstoffen, Papier, Karton, Anstrichfilmen, Holz u.ä. verwendet werden.

Mikroorganismen, die einen Abbau oder eine Veränderung der technischen Materialien bewirken können, sind beispielsweise Bakterien, Pilze, Hefen, Algen und Schleimorganismen. Vorzugsweise wirken die erfindungsgemässen Substanzen gegen Pilze und Schleimorganismen; von der fungiziden Wirkung werden Schimmelpilze ebenso erfasst wie Holzzerstörende und Holzverfärbende Pilze.

Es seien beispielsweise Mikroorganismen der folgenden Gattungen genannt:

Alternaria, wie Alternaria tenuis,
Alternaria, wie Alternaria speciales,
Aspergillus, wie Aspergillus niger,
Chaetomium, wie Chaetomium globosum,
Coniophora, wie Coniophora cerebella,
Lentinus, wie Lentinus tigrinus,
Penicillium, wie Penicillium glaucum,
Penicillium, wie Penicillium citrinum,
Polyporus, wie Polyporus versicolor,
Aureo basidium, wie Aureobasidium pullulans,
Sclerophoma, wie Sclerophoma pityophila,
Stachybotrys, wie Stachybotrys atra,
Paecilomyces, wie Paecilomyces varioti,
Cladosporium, wie Cladosporium herbarum,
Aspergillus, wie Aspergillus ustus,
Aspergillus, wie Aspergillus flavus.

Je nach ihrem Anwendungsgebiet können die erfindungsgemässen Substanzen in die üblichen Formulierungen überführt werden, wie Lösungen, Emulsionen, Suspensionen, Pulver, Pasten und Granulate. Diese können in an sich bekannter Weise hergestellt werden, z.B. durch Vermischen der Wirkstoffe mit einem Streckmittel, das aus flüssigem Lösungsmittel und/oder festen Trägerstoffen besteht, gegebenenfalls unter Verwendung von oberflächenaktiven Mitteln, wie Emulgatoren und/oder Dispergiermitteln, wobei beispielsweise im Falle der Benutzung von Wasser als Streckmittel, gegebenenfalls organische Solventien, als Hilfslösungsmittel verwendet werden können.

Organische Solventien für die Wirkstoffe können beispielsweise Alkohole, wie niedere Alkohole, vorzugsweise Ethanol, Isopropanol oder Benzylalkohol, Ketone, wie Aceton oder Methylethylketon, flüssige Kohlenwasserstoffe, wie Benzinfraktionen, chlorierte Kohlenwasserstoffe, wie 1,2-Dichlorethan, sein.

Die erfindungsgemässen mikrobiziden Mittel enthalten im allgemeinen 10 bis 100 Gew.-%, bevorzugt 50 bis 80 Gew.-%, der Propargylsulfamide als Wirkstoff.

Die Anwendungskonzentration der erfindungsgemässen Substanzen richtet sich nach der Art und dem Vorkommen der zu bekämpfenden Mikroorganismen, sowie nach der Zusammensetzung des zu schützenden Materials. Die optimale Einsatzmenge kann durch Testreihen ermittelt werden. Im allgemeinen liegen die Anwendungskonzentrationen im Bereich von 0,001 bis 5 Gew.-%, vorzugsweise von 0,05 bis 1,0 Gew.-%, bezogen auf das zu schützende Material.

Die erfindungsgemässen neuen Wirkstoffe können bei der Anwendung im Materialschutz auch in Mischung mit anderen bekannten Wirkstoffen, insbesondere zur Verbreiterung des Wirkungsspektrums mit Bakteriziden, angewendet werden; genannt seien beispielsweise Phenolderivate, Formaldehydabspaltende Verbindungen, Dithiocarbamate, Benzimidazolylcarbamate, Thiazolylbenzimidazol, Isothiazolon- und Benzisothialzolonderivate, Trihalogenmethylthioverbin-

dungen, Tetrachlorisophthalsäuredinitril, Mercaptobenzthiazol, Mercaptopyridin.

Im Gegensatz zu bekannten mikrobizid wirksamen Sulfamiden, genannt sei beispielsweise N,N-Dimethyl-N'-phenyl-N'-fluordichlormethylthio-sulfamid, zeichnen sich die erfindungsgemässen N-Jod-propargylsulfamide durch Stabilität in alkalischen Medien aus, wodurch ihre Verwendbarkeit als Mikrobizide zum Schutze technischer Materialien wesentlich verbreitert wird.

Herstellungsbeispiele

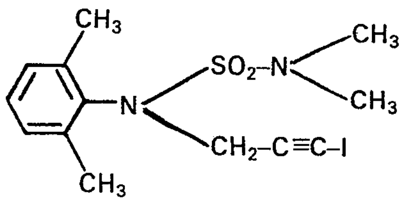

Beispiel 1

a) 26,6 g (0,1 Mol) N', N'-Dimethyl-N-propargyl-2,6-dimethylsulfanilid werden in 200 ml Methanol gelöst, und die Lösung wird auf −5 °C abgekühlt. 75 g 25%ige Natronlauge werden so zugetropft, dass die Innentemperatur 0 °C nicht übersteigt; anschliessend werden bei 0–5 °C 34 g fein gepulvertes Jod eingetragen und es wird 30 min. bei 0–5 °C nachgerührt. Man lässt 250 ml Wasser unter starkem Rühren langsam einfliessen, gibt etwas 10%ige Bisulfitlösung bis zur Entfärbung zu und saugt das abgeschiedene Produkt ab. Man erhält 28 g (71,4% der Theorie) N', N'-Dimethyl-N- (3-jod-propargyl)-2,6- dimethylsulfanilid als farblose Kristalle vom Schmelzpunkt 119–121 °C (aus Cyclohexan).

Das als Ausgangsstoff eingesetzte Propargylanilid wird folgendermassen hergestellt:

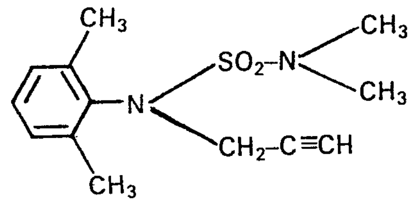

b) Eine Lösung von 17,5 g (0,155 Mol) Kalium-tert.-butylat in 50 ml Tetrahydrofuran wird zu einer Lösung von 34,3 g (0,15 Mol) N', N'-Dimethyl-2,6-dimethylsulfanilid in 35 ml Tetrahydrofuran unter Kühlung zugegeben. Das Kaliumsalz scheidet sich als viskose Masse ab, die nach kurzem Stehen durchkristallisiert; es wird abgesaugt, mit Ether gewaschen und dann bei 0–5 °C unter Rühren in eine Lösung von 13 g (0,17 Mol) 3-Chlorpropin in 100 ml Dimethylformamid eingetragen. Anschliessend wird langsam auf 70 °C erwärmt und 3 Stunden bei dieser Temperatur gehalten. Das Lösungsmittel wird im Wasserstrahlvakuum bei 70 °C entfernt und der Rückstand auf Wasser gegossen. Der abgeschiedene Feststoff wird abgesaugt und im Exsiccator getrocknet. Man erhält 36,5 g (91,5% der Theorie) N', N'-Dimethyl-N-propargyl-2,6-dimethylsulfanilid vom Schmelzpunkt 109–11 °C (aus Cyclohexan).

Analog wie unter Beispiel 1 beschrieben, können die erfindungsgemässen Verbindungen der Formel (I)

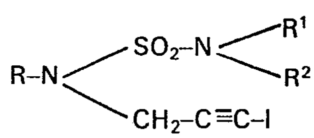 (I)

hergestellt werden:

| Beispiel Nr. | R | $R^1$ | $R^2$ | Schmelzpunkt ( °C); Brechungsindex $[n_D^{20}:]$ |
|---|---|---|---|---|
| 2 | 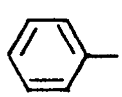 | $CH_3$ | $CH_3$ | 74–75 |
| 3 | 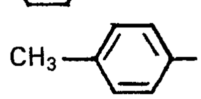 | $CH_3$ | $CH_3$ | 78–79 |
| 4 | 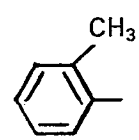 | $CH_3$ | $CH_3$ | 75 |
| 5 | 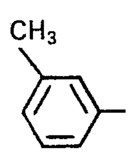 | $CH_3$ | $CH_3$ | 1,5700 |

| Beispiel Nr. | R | $R^1$ | $R^2$ | Schmelzpunkt (°C); Brechungsindex [$n_D^{20}$:] |
|---|---|---|---|---|
| 6 | 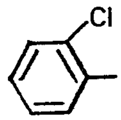 | $CH_3$ | $CH_3$ | 82–85 |
| 7 | 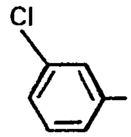 | $CH_3$ | $CH_3$ | 55–57 |
| 8 | 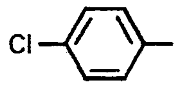 | $CH_3$ | $CH_3$ | 84–85 |
| 9 | 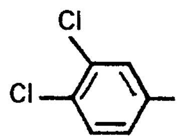 | $CH_3$ | $CH_3$ | 1,5814 |
| 10 | 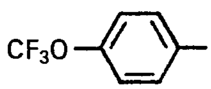 | $CH_3$ | $CH_3$ | zähes Öl |
| 11 | 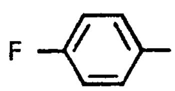 | $CH_3$ | $CH_3$ | 67 |
| 12 | 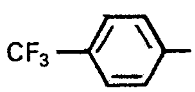 | $CH_3$ | $CH_3$ | 1,5405 |
| 13 | 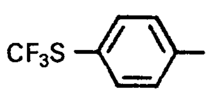 | $CH_3$ | $CH_3$ | 1,5000 |
| 14 | $CH_3$ | $CH_3$ | $CH_3$ | 36 |
| 15 | $C_2H_5$ | $CH_3$ | $CH_3$ | 48 |
| 16 | i–$C_3H_7$ | $CH_3$ | $CH_3$ | 85,5 |
| 17 | t–$C_4H_9$ | $CH_3$ | $CH_3$ | 65–8 |
| 18 | n–$C_{12}H_{25}$ | $CH_3$ | $CH_3$ | 27 |
| 19 | 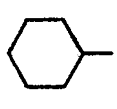 | $CH_3$ | $CH_3$ | 75–7 |
| 20 | 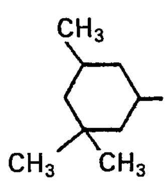 | $CH_3$ | $CH_3$ | 85–8 |
| 21 | 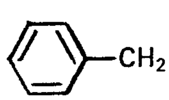 | $CH_3$ | $CH_3$ | 30 |

| Beispiel Nr. | R | $R^1$ | $R^2$ | Schmelzpunkt (°C); Brechungsindex $[n_D^{20}:]$ |
|---|---|---|---|---|
| 22 | 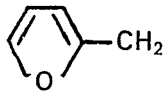 | $CH_3$ | $CH_3$ | 1,5411 |
| 23 | H | $-(CH_2)_2-O-(CH_2)_2-$ | | 74–6 |
| 24 | 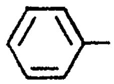 | $-(CH_2)_2-O-(CH_2)_2-$ | | 1,5665 |
| 25 | 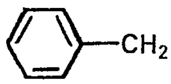 | $-(CH_2)_2-O-(CH_2)_2-$ | | 120–21 |
| 26 | 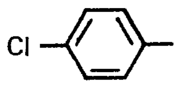 | $-(CH_2)_2-O-(CH_2)_2-$ | | 60–2 |
| 27 | 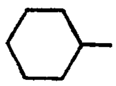 | $-(CH_2)_2-O-(CH_2)_2-$ | | 120–2 |
| 28 | $CH_3$ | $-(CH_2)_2-O-(CH_2)_2-$ | | 64–6 |
| 29 | 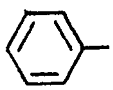 | $-(CH_2)_5-$ | | 60–2 |
| 30 | 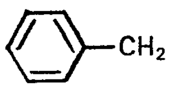 | $-(CH_2)_5-$ | | 65–8 |
| 31 | 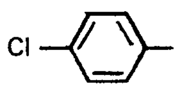 | $-(CH_2)_5-$ | | 1,5728 |
| 32 | $CH_3$ | $-(CH_2)_4-$ | | 82–5 |
| 33 | 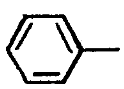 | $-(CH_2)_4-$ | | 75–7 |
| 34 | 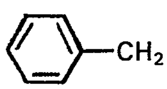 | $-(CH_2)_4-$ | | 108–10 |
| 35 | 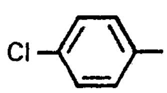 | $-(CH_2)_4-$ | | 92–3 |
| 36 | 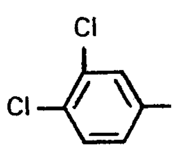 | $CH_3$ | $CH_3$ | 1,5778 |

Anwendungsbeispiele

In den nachfolgenden Beispielen werden die hier angegebenen, bekannten Verbindungen als Vergleichssubstanzen eingesetzt:

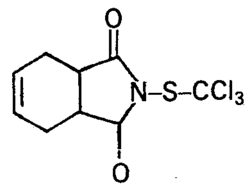

(A) N-Trichlormethylthio-tetrahydrophthalimid

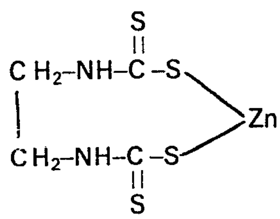

(B) Zink-ethylen-1,2-bis-(dithiocarbamat)

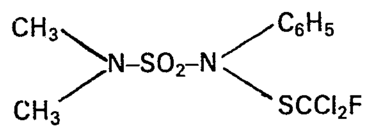

(D) N,N-Dimethyl-N'-phenyl-N'-fluordichlormethylthio-sulfamid

Beispiel A

Venturia-Test (Apfel) / protektiv

Lösungsmittel: 4,7 Gewichtsteile Aceton

Emulgator: 0,3 Gewichtsteile Alkyl-aryl-polyglykolether

Zur Herstellung einer zweckmässigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wässrigen Konidiensuspension des Apfelschorferregers (Venturia inaequalis) inokuliert und verbleiben dann 1 Tag bei 20 °C und 100% relativer Luftfeuchtigkeit in einer Inkubationskabine.

Die Pflanzen werden dann im Gewächshaus bei 20 °C und einer relativen Luftfeuchtigkeit von ca. 70% aufgestellt.

12 Tage nach der Inokulation erfolgt die Auswertung.

Eine deutliche Überlegenheit in der Wirksamkeit gegenüber der Verbindung (A) aus dem Stand der Technik zeigen in diesem Test z.B. die Verbindungen gemäss der Herstellungsbeispiele: 3, 5, 1, 8, 9, 33, 35, 4 und 6.

Beispiel B

Phytophthora-Test (Tomate) / protektiv

Lösungsmittel: 4,7 Gewichtsteile Aceton

Emulgator: 0,3 Gewichtsteile Alkyl-aryl-polyglykolether

Zur Herstellung einer zweckmässigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wässrigen Sporensuspension von Phytophthora infestans inokuliert.

Die Pflanzen werden in einer Inkubationskabine mit 100% relativer Luftfeuchtigkeit und ca. 20 °C aufgestellt.

3 Tage nach der Inokulation erfolgt die Auswertung.

Eine deutliche Überlegenheit in der Wirksamkeit gegenüber dem Stand der Technik zeigen in diesem Test z.B. die Verbindungen gemäss folgender Herstellungsbeispiele: 5, 8, 29, 4 und 6.

Beispiel C

Puccinia-Test (Weizen) / protektiv

Lösungsmittel: 100 Gewichtsteile Dimethylformamid

Emulgator: 0,25 Gewichtsteile Alkylarylpolyglykolether

Zur Herstellung einer zweckmässigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit einer Sporensuspension von Puccinia recondita in einer 0,1%igen wässrigen

Agarlösung inokuliert. Nach Antrocknen besprüht man die Pflanzen mit der Wirkstoffzubereitung taufeucht. Die Pflanzen verbleiben 24 Stunden bei 20 °C und 100% rel. Luftfeuchtigkeit in einer Inkubationskabine.

Die Pflanzen werden in einem Gewächshaus bei einer Temperatur von ca. 20 °C und einer relativen Luftfeuchtigkeit von ca. 80% aufgestellt, um die Entwicklung von Rostpusteln zu begünstigen.

10 Tage nach der Inokulation erfolgt die Auswertung.

Eine deutliche Überlegenheit in der Wirksamkeit gegenüber dem Stand der Technik zeigen bei diesem Test z.B. die Verbindungen gemäss folgender Herstellungsbeispiele: 2, 3, 11, 10, 12, 13, 19, 28, 32, 31 und 4.

Beispiel D

Pyricularia-Test (Reis) / protektiv

Lösungsmittel: 12,5 Gewichtsteile Aceton

Emulgator: 0,3 Gewichtsteile Alkylarylpolyglykolether

Zur Herstellung einer zweckmässigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wässrigen Sporensuspension von Pyricularia oryzae inokuliert. Anschliessend werden die Pflanzen in einem Gewächshaus bei 100% rel. Luftfeuchtigkeit und 25 °C aufgestellt.

4 Tage nach der Inokulation erfolgt die Auswertung des Krankheitsbefalls.

Eine deutliche Überlegenheit in der Wirksamkeit gegenüber dem Stand der Technik zeigen bei diesem Test z.B. die Verbindungen gemäss folgender Herstellungsbeispiele: 3, 29 und 30.

Beispiel E

Pyricularia-Test (Reis) / systemisch

Lösungsmittel: 12,5 Gewichtsteile Aceton

Emulgator: 0,3 Gewichtsteile Alkylarylpolyglykolether

Zur Herstellung einer zweckmässigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf systemische Eigenschaften werden 40 ml der Wirkstoffzubereitung auf Einheitserde gegossen, in der junge Reispflanzen angezogen wurden. 7 Tage nach der Behandlung werden die Pflanzen mit einer wässrigen Sporensuspension von Pyricularia oryzae inokuliert. Danach verbleiben die Pflanzen in einem Gewächshaus bei einer Temperatur von 25 °C und einer rel. Luftfeuchtigkeit von 100% bis zur Auswertung.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäss folgender Herstellungsbeispiele: 26, 28 und 11.

Beispiel F

Wirkung gegen Pilze

In einen Agar, der aus Bierwürze und Pepton hergestellt wurde, werden die erfindungsgemässen Verbindungen in abgestuften Konzentrationen zwischen 0,1 bis 5000 mg/l Versuchsprobe eingearbeitet. Nach dem Erstarren des Agars erfolgt die Kontamination der so hergestellten Agarproben mit Reinkulturen verschiedener Testorganismen.

Nach zweiwöchiger Lagerung bei 28 °C und 60 bis 70% relativer Luftfeuchtigkeit wird ausgewertet. Die minimale Hemmkonzentration (MHK), d.h. die geringste in einer Agarprobe enthaltene Konzentration der Substanz, bei der keinerlei Bewuchs durch die verwendete Art erfolgt, wird bestimmt.

In diesem Test zeigen eine sehr gute Wirkung die Verbindungen gemäss Herstellungsbeispielen: 2, 6, 8, 5, 14, 15, 16, 17, 19 und 22.

Beispiel G

Wirkung gegen Schleimorganismen

Erfindungsgemässe Verbindungen werden in Konzentrationen von jeweils 0,1 bis 100 mg/l in Allens Nährlösung (Arch. Mikrobiol. 17, 34 bis 53 (1952) ), die in 4 l sterilem Wasser, 0,2 g Ammoniumchlorid, 4,0 g Natriumnitrat, 1,0 g Dikaliumhydrogenphosphat, 0,2 g Calciumchlorid, 2,05 g Magnesiumsulfat, 0,02 g Eisenchlorid und 1% Caprolactam enthält, in wenig Aceton gelöst, zur Anwendung gebracht. Kurz vorher wird die Nährlösung mit Schleimorganismen (ca. $10^6$ Keime/ml), die aus bei der Polyamid-Herstellung verwendeten Spinnwasser-Kreisläufen isoliert wurden, infiziert. Nährlösungen, die die minimale Hemmkonzentration (MHK) oder grössere Wirkstoffkonzentrationen aufweisen, sind nach 3wöchiger Kultur bei Raumtemperatur noch völlig klar, d.h. die in wirkstofffreien Nährlösungen nach 3 bis 4 Tagen bemerkbare starke Vermehrung der Mikroben und Schleimbildung unterbleibt.

In diesem Test wurden die Verbindungen gemäss Herstellungsbeispielen eingesetzt: 2, 6, 8, 5, 14, 15 und 22.

Beispiel H

Prüfung von Anstrichen auf Schimmelfestigkeit

Die Prüfung wird in Anlehnung an den Report 219 der Defense Standards Laboratories Maibyrnong/Australien wie folgt durchgeführt: Ein glatter Karton wird beidseitig mit dem zu prüfenden Produkt bestrichen und 8 Tage bei Raumtemperatur getrocknet. Zur Alterung wird ein Teil des Anstriches 24 Stunden in fliessendem Wasser von 24 °C gewässert oder 8 Tage mit Frischluft von 40 bis 60 °C belüftet oder 110 Stunden einem trockenen Xenon-Test ausgesetzt. Von den so vorbereiteten Proben werden Abschnitte von 5 × 5 cm einzeln in Petrischalen auf einen Traubenzucker-Nährboden aufgelegt und mit einer Sporensuspension der nachstehenden Pilze kontaminiert: Aspergillus niger, Pullularia pullulans, Alternaria speciales, Penicillium citrinum, Stachybotrys atra, Paecilomyces varioti, Cladosporium herbarum, Aspergillus ustus und Aspergillus flavus.

Die kontaminierten Schalen werden bei 28 bis 30 °C und 90 bis 95% relativer Luftfeuchtigkeit gelagert und nach drei Wochen ausgewertet. Anstriche gelten als schimmelfest, wenn die Proben nach diesen Tests pilzfrei bleiben.

Nach der oben angegebenen Testmethode wird eine handelsübliche, alkalisch reagierende Dispersionsfarbe auf Polyvinylacetat-Basis auf Schimmelfestigkeit geprüft.

Proben des Anstrichmittels, die bezogen auf Gesamtfeststoffgehalt 1,5–2% (oder mehr) N,N-Dimethyl-N'-phenyl-N'-jodpropargyl-sulfamid (2) enthalten, liefern sehr gute schimmelfeste Anstriche; auch dann, wenn die Anstriche zuvor den obengenannten Belastungen unterzogen wurden.

## Patentansprüche

1. Jodpropargylsulfamide der Formel (I)

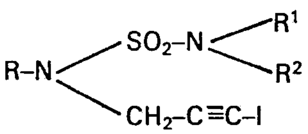

(I)

in welcher

R für Wasserstoff, für geradkettiges oder verzweigtes Alkyl mit 1 bis 18 Kohlenstoffatomen, für gegebenenfalls ein- bis fünffach durch Alkyl mit 1 bis 6 Kohlenstoffatomen substituiertes Cycloalkyl mit 3 bis 8 Ringgliedern, für gegebenenfalls ein- bis fünffach, gleich oder verschieden durch Alkyl mit 1 bis 4 Kohlenstoffatomen und/oder Halogen am Phenylring substituiertes Phenylalkyl mit 1 bis 4 Kohlenstoffatomen im Alkylteil, für gegebenenfalls ein- bis vierfach gleich oder verschieden durch Alkyl mit 1 bis 4 Kohlenstoffatomen und/oder Halogen substituiertes Heterocyclylalkyl mit 1 bis 4 Kohlenstoffatomen im Alkylteil und mit 5 bis 8 Ringgliedern im Heterocyclylteil, wobei der Heterocyclylteil 1 bis 3 Heteroatome, wie Stickstoff, Sauerstoff und/oder Schwefel, enthalten kann, ferner für Phenyl, das ein- bis fünffach, gleich oder verschieden durch Halogen, Cyano, Nitro, Alkyl mit 1 bis 5 Kohlenstoffatomen, Alkoxy mit 1 bis 5 Kohlenstoffatomen, Alkylthio mit 1 bis 5 Kohlenstoffatomen, Alkoxycarbonyl mit 1 bis 5 Kohlenstoffatomen im Alkoxyteil, Halogenalkyl oder Halogenalkoxy bzw. Halogenalkylthio oder Halogenalkylsulfonyl mit 1 bis 5 Kohlenstoffatomen und 1 bis 5 Halogenatomen je Halogenalkylrest substituiert sein kann, steht,

$R^1$ und $R^2$ für gleiches oder verschiedenes Alkyl mit je 1 bis 5 Kohlenstoffatomen oder

$R^1$ und $R^2$ für eine Alkylenbrücke mit 2 bis 8 Kohlenstoffatomen stehen, die mit dem Stickstoff, an dem sie stehen, einen Ring bilden, der durch weitere Heteroatome wie Sauerstoff, Schwefel, die Gruppen SO bzw. $SO_2$ oder durch N-Alkyl mit 1 bis 3 Kohlenstoffatomen im Alkylteil unterbrochen sein kann. Die Ringe können gegebenenfalls ein- bis fünffach durch Alkyl mit 1 bis 6 Kohlenstoffatomen substituiert sein.

2. Jodpropargylsulfamide gemäss Anspruch 1, wobei in der Formel (I)

R für Wasserstoff, geradkettiges oder verzweigtes Alkyl mit 1 bis 12 Kohlenstoffatomen, für gegebenenfalls ein- bis dreifach durch Alkyl mit 1 bis 4 Kohlenstoffatomen substituiertes Cycloalkyl mit 3 bis 6 Ringgliedern, für gegebenenfalls ein- bis fünffach, gleich oder verschieden durch Alkyl mit 1 bis 3 Kohlenstoffatomen und/oder Fluor, Chlor, Brom oder Jod am Phenylring substituiertes Phenylalkyl mit 1 oder 2 Kohlenstoffatomen im Alkylteil, für gegebenenfalls ein- bis dreifach, gleich oder verschieden durch Methyl, Ethyl, Fluor, Chlor oder Brom substituiertes Heterocyclylalkyl mit 1 oder 2 Kohlenstoffatomen im Alkylteil und mit 5 oder 6 Ringgliedern im Heterocyclylteil, wobei der Heterocyclylteil 1 oder 2 gleiche oder verschiedene Heteroatome, wie Stickstoff oder Sauerstoff, enthalten kann, ferner für Phenyl, das ein- bis dreifach, gleich oder verschieden durch Fluor, Chlor, Brom, Cyano, Nitro, Alkyl mit 1 bis 3 Kohlenstoffatomen, Methoxy, Ethoxy, Trifluormethyl, Trifluormethoxy, Trifluormethylthio, Difluorchlormethyl, Difluorchlormethoxy, Difluorchlormethylthio, Trifluormethylsulfonyl, Methoxycarbonyl oder Ethoxycarbonyl substituiert sein kann, steht und

$R^1$ und $R^2$ gleich oder verschieden sind und für Alkyl mit 1 bis 3 Kohlenstoffatomen stehen oder

$R^1$ und $R^2$ für eine Alkylenbrücke mit 2 bis 5 Kohlenstoffatomen stehen, die mit dem Stickstoffatom, an dem sie stehen, einen Ring bilden, der durch Sauerstoffatome, Schwefel oder die N-Alkyl-Gruppe, die 1 bis 3 Kohlenstoffatome enthält, unterbrochen sein kann.

3. Jodpropargylsulfamide gemäss Anspruch 1, wobei in der Formel (I)

R für Wasserstoff, für Alkyl mit 1 bis 12 Kohlenstoffatomen, für gegebenenfalls ein- bis dreifach durch Methyl substituiertes Cyclopentyl oder Cyclohexyl, für Benzyl, für Furfuryl, für gegebenenfalls ein- oder zweifach, gleich oder verschieden durch Methyl, Chlor, Fluor, Trifluormethoxy, Trifluormethyl und Trifluormethylthio substituiertes Phenyl steht,

$R^1$ und $R^2$ für Methyl stehen oder

$R^1$ und $R^2$ für eine Alkylenbrücke mit 4 oder 5 Kohlenstoffatomen stehen, die mit dem Stickstoffatom, an dem sie stehen, einen Ring bilden, der gegebenenfalls durch Sauerstoff unterbrochen sein kann.

4. Verfahren zur Herstellung von Jodpropargylsulfamiden der Formel (I)

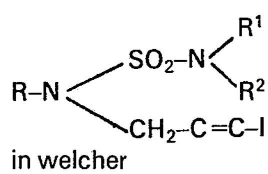

(I)

in welcher

R für Wasserstoff, für geradkettiges oder verzweigtes Alkyl mit 1 bis 18 Kohlenstoffatomen, für gegebenenfalls ein- bis fünffach durch Alkyl mit 1 bis 6 Kohlenstoffatomen substituiertes Cycloalkyl mit 3 bis 8 Ringgliedern, für gegebenenfalls ein- bis fünffach, gleich oder verschieden durch Alkyl mit 1 bis 4 Kohlenstoffatomen und/oder Halogen am Phenylring substituiertes Phenylalkyl mit 1 bis 4 Kohlenstoffatomen im Alkylteil, für gegebenenfalls ein- bis vierfach gleich oder verschieden durch Alkyl mit 1 bis 4 Kohlenstoffatomen und/oder Halogen substituiertes Heterocyclylalkyl mit 1 bis 4 Kohlenstoffatomen im Alkylteil und mit 5 bis 8 Ringgliedern im Heterocyclylteil, wobei der Heterocyclylteil 1 bis 3 Heteroatome, wie Stickstoff, Sauerstoff und/oder Schwefel, enthalten kann, ferner für Phenyl, das ein- bis fünffach, gleich oder verschieden durch Halogen, Cyano, Nitro, Alkyl mit 1 bis 5 Kohlenstoffatomen, Alkoxy mit 1 bis 5 Kohlenstoffatomen, Alkylthio mit 1 bis 5 Kohlenstoffatomen, Alkoxycarbonyl mit 1 bis 5 Kohlenstoffatomen im Alkoxyteil, Halogenalkyl oder Halogenalkoxy bzw. Halogenalkylthio oder Halogenalkylsulfonyl mit 1 bis 5 Kohlenstoffatomen und 1 bis 5 Halogenatomen je Halogenalkylrest substituiert sein kann, steht,

$R^1$ und $R^2$ für gleiches oder verschiedenes Alkyl mit je 1 bis 5 Kohlenstoffatomen oder

$R^1$ und $R^2$ für eine Alkylenbrücke mit 2 bis 8 Kohlenstoffatomen stehen, die mit dem Stickstoff, an dem sie stehen, einen Ring bilden, der durch weitere Heteroatome wie Sauerstoff, Schwefel, die Gruppen SO bzw. $SO_2$ oder durch N-Alkyl mit 1 bis 3 Kohlenstoffatomen im Alkylteil unterbrochen sein kann. Die Ringe können gegebenenfalls ein- bis fünffach durch Alkyl mit 1 bis 6 Kohlenstoffatomen substituiert sein, dadurch gekennzeichnet, dass man Propargylsulfamide der Formel (II)

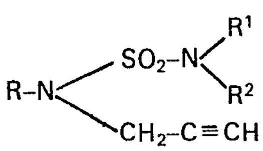

(II)

in welcher

R, $R^1$ und $R^2$ die oben angegebene Bedeutung haben,

in Gegenwart einer Base gegebenenfalls in Gegenwart eines Lösungs- oder Verdünnungsmittels mit Jod umsetzt.

5. Mikrobizide Mittel, gekennzeichnet durch einen Gehalt an mindestens einem Jodpropargylsulfamid der Formel (I).

6. Verwendung von Jodpropargylsulfamiden der Formel (I) zur Bekämpfung von Mikroorganismen.

7. Verfahren zur Bekämpfung von Mikroorganismen, dadurch gekennzeichnet, dass man Jodpropargylsulfamide der Formel (I) auf Mikroorganismen und/oder ihren Lebensraum einwirken lässt.

8. Verfahren zur Herstellung von mikrobiziden Mitteln, dadurch gekennzeichnet, dass man Jodpropargylsulfamide der Formel (I) mit Streckmitteln und/oder oberflächenaktiven Mitteln vermischt.

**Revendications**

1. Iodopropargylsulfamides de formule (I):

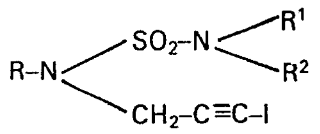

(I)

dans laquelle:

R représente un atome d'hydrogène, un groupe alkyle linéaire ou ramifié ayant 1 à 18 atomes de carbone, un groupe cycloalkyle ayant 3 à 8 chaînons de cycle et éventuellement substitué 1 à 5 fois par un radical alkyle ayant 1 à 6 atomes de carbone, un groupe phénylalkyle ayant 1 à 4 atomes de carbone dans la partie alkyle et éventuellement substitué 1 à 5 fois, de manière identique ou différente, par un radical alkyle ayant 1 à 4 atomes de carbone et/ou de l'halogène sur le noyau phényle, un groupe hétérocyclylalkyle ayant 1 à 4 atomes de carbone dans la partie alkyle et 5 à 8 chaînons de cycle dans la partie hétérocyclique, qui est éventuellement substitué 1 à 4 fois, de façon identique ou différente, par un radical alkyle ayant 1 à 4 atomes de carbone et/ou par de l'halogène, la partie hétérocyclique pouvant contenir 1 à 3 hétéroatomes comme l'azote, l'oxygène et/ou le soufre, ainsi qu'un groupe phényle qui peut être substitué 1 à 5 fois, de façon identique ou différente, par de l'halogène, par un radical cyano, nitro, alkyle ayant 1 à 5 atomes de carbone, alcoxy ayant 1 à 5 atomes de carbone, alkylthio ayant 1 à 5 atomes de carbone, alcoxycarbonyle ayant 1 à 5 atomes de carbone dans la partie alcoxy, halogénoalkyle ou halogénoalcoxy ou halogénoalkylthio ou halogènealkylsulfonyle ayant 1 à 5 atomes de carbone et 1 à 5 atomes d'halogène par reste halogénoalkyle,

$R^1$ et $R^2$ représentent un groupe alkyle, identique ou différent, ayant chacun 1 à 5 atomes de carbone, ou

$R^1$ et $R^2$ représentent un pont alkylène ayant 2 à 8 atomes de carbone et qui, avec l'atome d'azote auquel ils sont fixés, forment un noyau qui peut être interrompu par d'autres hétéroatomes comme l'oxygène, le soufre, les groupes SO ou $SO_2$ ou par un radical N-alkyle ayant 1 à 3 atomes de carbone dans la partie alkyle. Les noyaux peuvent éventuellement être substitués 1 à 5 fois par un radical alkyle ayant 1 à 6 atomes de carbone.

2. Iodopropargylsulfamides selon la revendication 1, dans lesquels, dans la formule (I):

R représente un atome d'hydrogène, un groupe alkyle linéaire ou ramifié ayant 1 à 12 atomes de carbone, un groupe cycloalkyle ayant 3 à 6 chaînons cycliques et qui est éventuellement substitué 1 à 3 fois par un radical alkyle ayant 1 à 4 atomes de carbone, un groupe phénylalkyle ayant 1 ou 2 atomes de carbone dans la partie alkyle et qui

est éventuellement substitué 1 à 5 fois, de façon identique ou différente, par un radical alkyle ayant 1 à 3 atomes de carbone et/ou par du fluor, du chlore, du brome ou de l'iode sur le noyau phényle, un groupe hétérocyclylalkyle ayant 1 ou 2 atomes de carbone dans la partie alkyle et 5 ou 6 chaînons de cycle dans la partie hétérocyclique, et qui est éventuellement substitué 1 à 3 fois, de façon identique ou différente, par un radical méthyle, éthyle, fluoro, chloro ou bromo, la partie hétérocyclique pouvant contenir 1 ou 2 hétéroatomes identiques ou différents, comme l'azote ou le soufre, un groupe phényle, qui peut être substitué 1 à 3 fois, de façon identique ou différente, par un atome de fluor, de chlore, de brome, un radical cyano, nitro, alkyle ayant 1 à 3 atomes de carbone, méthoxy, éthoxy, trifluorométhyle, trifluorométhoxy, trifluorométhylthio, difluorochlorométhyle, difluorochlorométhoxy, difluorochlorométhylthio, trifluorométhylsulfonyle, méthoxycarbonyle ou éthoxycarbonyle, et

$R^1$ et $R^2$ sont identiques ou différents et représentent chacun un groupe alkyle ayant 1 à 3 atomes de carbone ou

$R^1$ et $R^2$ représentent un pont alkylène comportant 2 à 5 atomes de carbone et qui forme, avec l'atome d'azote auquel il est relié, un noyau qui peut être interrompu par des atomes d'oxygène, du soufre ou le groupe N-alkyle qui contient 1 à 3 atomes de carbone.

3. Iodopropargylsulfamides selon la revendication 1, dans lesquels, dans la formule (I),

R représente un atome d'hydrogène, un groupe alkyle ayant 1 à 12 atomes de carbone, un groupe cyclopentyle ou cyclohexyle éventuellement substitué 1 à 3 fois par un radical méthyle, un groupe benzyle, un groupe furfuryle, un groupe phényle éventuellement substitué 1 ou 2 fois, de façon identique ou différente, par un radical méthyle, chloro, fluoro, trifluorométhoxy, trifluorométhyle et trifluorométhylthio,

$R^1$ et $R^2$ représentent chacun un groupe méthyle, ou

$R^1$ et $R^2$ représentent un pont alkylène ayant 4 ou 5 atomes de carbone et qui forme, avec l'atome d'azote auquel il est fixé, un noyau pouvant être interrompu éventuellement par de l'oxygène.

4. Procédé pour préparer des iodopropargylsulfamides de formule (I):

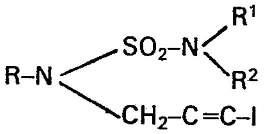

(I)

dans laquelle:

R représente un atome d'hydrogène, un groupe alkyle linéaire ou ramifié ayant 1 à 18 atomes de carbone, un groupe cycloalkyle ayant 3 à 8 chaînons de cycle et éventuellement substitué 1 à 5 fois par un radical alkyle ayant 1 à 6 atomes de carbone, un groupe phénylalkyle ayant 1 à 4 atomes de carbone dans la partie alkyle et éventuellement substitué 1 à 5 fois, de manière identique ou différente, par un radical alkyle ayant 1 à 4 atomes de carbone et/ou de l'halogène sur le noyau phényle, un groupe hétérocyclylalkyle ayant 1 à 4 atomes de carbone dans la partie alkyle et 5 à 8 chaînons de cycle dans la partie hétérocyclique, qui est éventuellement substitué 1 à 4 fois, de façon identique ou différente, par un radical alkyle ayant 1 à 4 atomes de carbone et/ou par de l'halogène, la partie hétérocyclique pouvant contenir 1 à 3 hétéroatomes comme l'azote, l'oxygène et/ou le soufre, ainsi qu'un groupe phényle qui peut être substitué 1 à 5 fois, de façon identique ou différente, par de l'halogène, par un radical cyano, nitro, alkyle ayant de 1 à 5 atomes de carbone, alcoxy ayant 1 à 5 atomes de carbone, alkylthio ayant 1 à 5 atomes de carbone, alcoxycarbonyle ayant 1 à 5 atomes de carbone dans la partie alcoxy, halogénoalkyle ou halogénoalcoxy ou halogénoalkylthio ou halogènealkylsulfonyle ayant 1 à 5 atomes de carbone et 1 à 5 atomes d'halogène par reste halogénoalkyle,

$R^1$ et $R^2$ représentent un groupe alkyle, identique ou différent, ayant chacun 1 à 5 atomes de carbone, ou

$R^1$ et $R^2$ représentent un pont alkylène ayant 2 à 8 atomes de carbone et qui, avec l'atome d'azote auquel ils sont fixés forment un noyau qui peut être interrompu par d'autres hétéro-atomes comme l'oxygène, le soufre, les groupes SO ou $SO_2$ ou par un radical N-alkyle ayant 1 à 3 atomes de carbone dans la partie alkyle, les noyaux pouvant éventuellement être substitués 1 à 5 fois par un radical alkyle ayant 1 à 6 atomes de carbone, caractérisé en ce qu'on fait réagir avec de l'iode, en présence d'une base et éventuellement en présence d'un solvant ou diluant, des propargylsulfamides de formule (II):

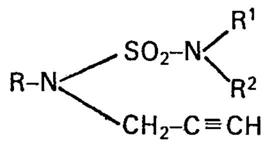

(II)

dans laquelle:

R, $R^1$ et $R^2$ ont le sens indiqué ci-dessus.

5. Agent microbicide, caractérisé en une teneur en au moins un iodopropargylsulfamide de formule (I).

6. Utilisation des iodopropargylsulfamides de formule (I) pour combattre les micro-organismes.

7. Procédé pour combattre les micro-organismes, caractérisé en ce qu'on fait agir des iodopropargylsulfamides de formule (I) sur les micro-organismes et/ou sur leur biotope

8. Procédé de préparation d'agents microbicides, caractérisé en ce qu'on mélange des iodopropargylsulfamides de formule (I) avec des agents d'allongement et/ou des agents tensioactifs.

## Claims

1. Iodopropargylsulphamides of the formula (I)

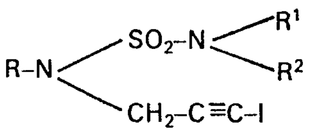

(I)

in which

R represents hydrogen, straight-chain or branched alkyl with 1 to 18 carbon atoms, cycloalkyl which has 3 to 8 ring members and is optionally mono- to pentasubstituted by alkyl with 1 to 6 carbon atoms, phenylalkyl which has 1 to 4 carbon atoms in the alkyl part and is optionally mono- to pentasubstituted in the phenyl ring by identical or different substituents from amongst alkyl with 1 to 4 carbon atoms and/or halogen, heterocyclylalkyl which has 1 to 4 carbon atoms in the alkyl part and 5 to 8 ring members in the heterocyclyl part and is optionally mono- to tetrasubstituted by identical or different substituents from amongst alkyl with 1 to 4 carbon atoms and/or halogen, it being possible for the heterocyclyl part to contain 1 to 3 hetero atoms such as nitrogen, oxygen and/or sulphur, also phenyl which can be mono- to pentasubstituted by identical or different substituents from amongst halogen, cyano, nitro, alkyl with 1 to 5 carbon atoms, alkoxy with 1 to 5 carbon atoms, alkylthio with 1 to 5 carbon atoms, alkoxycarbonyl with 1 to 5 carbon atoms in the alkoxy part, halogenoalkyl or halogenoalkoxy or halogenoalkylthio or halogenoalkylsulphonyl with 1 to 5 carbon atoms and 1 to 5 halogen atoms per halogenoalkyl radical,

$R^1$ and $R^2$ represent identical or different alkyl each with 1 to 5 carbon atoms or

$R^1$ and $R^2$ represent an alkylene bridge with 2 to 8 carbon atoms which, together with the nitrogen on which they are located, form a ring which can be interrupted by further hetero atoms such as oxygen, sulphur, the groups SO or $SO_2$ or by N-alkyl with 1 to 3 carbon atoms in the alkyl part. The rings can optionally be mono- to pentasubstituted by alkyl with 1 to 6 carbon atoms.

2. Iodopropargylsulphamides according to Claim 1, wherein, in formula (I)

R represents hydrogen, straight-chain or branched alkyl with 1 to 12 carbon atoms, cycloalkyl which has 3 to 6 ring members and is optionally mono- to trisubstituted by alkyl with 1 to 4 carbon atoms, phenylalkyl which has 1 or 2 carbon atoms in the alkyl part and is optionally mono- to pentasubstituted in the phenyl ring by identical or different substituents from amongst alkyl with 1 to 3 carbon atoms and/or fluorine, chlorine, bromine or iodine, heterocyclylalkyl which has 1 or 2 carbon atoms in the alkyl part and 5 or 6 ring members in the heterocyclyl part and is optionally mono- to tri-substituted by identical or different substituents from amongst methyl, ethyl, fluorine, chlorine or bromine, it being possible for the heterocyclyl part to contain 1 or 2 identical or different hetero atoms such as nitrogen or oxygen, also phenyl which can be mono-to tri-substituted by identical or different substituents from amongst fluorine, chlorine, bromine, cyano, nitro, alkyl with 1 to 3 carbon atoms, methoxy, ethoxy, trifluoromethyl, trifluoromethoxy, trifluoromethylthio, difluorochloromethyl, difluorochloromethoxy, difluorochloromethylthio, trifluoromethylsulphonyl, methoxycarbonyl or ethoxycarbonyl, and

$R^1$ and $R^2$ are identical or different and represent alkyl with 1 to 3 carbon atoms or

$R^1$ and $R^2$ represent an alkylene bridge with 2 to 5 carbon atoms which, together with the nitrogen atom on which they are located, form a ring which can be interrupted by oxygen atoms, sulphur or the N-alkyl group which contains 1 to 3 carbon atoms.

3. Iodopropargylsulphamides according to Claim 1, wherein in formula (I)

R represents hydrogen, alkyl with 1 to 12 carbon atoms, cyclohexyl or cyclopentyl optionally mono- to trisubstituted by methyl, benzyl, furfuryl, or phenyl which is optionally mono- or disubstituted by identical or different substituents from amongst methyl, chlorine, fluorine, trifluoromethoxy, trifluoromethyl and trifluoromethylthio,

$R^1$ and $R^2$ represent methyl or

$R^1$ and $R^2$ represent an alkylene bridge with 4 or 5 carbon atoms, which, together with the nitrogen atom on which they are located, form a ring which can optionally be interrupted by oxygen.

4. Process for the preparation of iodopropargylsulphamides of the formula (I)

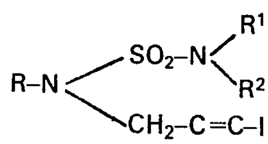

(I)

in which

R represents hydrogen, straight-chain or branched alkyl with 1 to 18 carbon atoms, cycloalkyl which has 3 to 8 ring members and is optionally mono- to pentasubstituted by alkyl with 1 to 6 carbon atoms, phenylalkyl which has 1 to 4 carbon atoms in the alkyl part and is optionally mono- to pentasubstituted in the phenyl ring by identical or different substituents from amongst alkyl with 1 to 4 carbon atoms and/or halogen, heterocyclylalkyl which has 1 to 4 carbon atoms in the alkyl part and 5 to 8 ring members in the heterocyclyl part and is optionally mono- to tetrasubstituted by identical or different substituents from amongst alkyl with 1 to 4 carbon atoms and/or halogen, it being possible for the heterocyclyl part to contain 1 to 3 hetero atoms such as nitrogen, oxygen and/or sulphur, also phenyl which can be mono- to pentasubstituted by identical or different substituents from amongst halogen, cyano, nitro, alkyl with 1 to 5 carbon atoms, alkoxy with 1 to 5 carbon atoms, alkylthio with 1 to 5 carbon atoms, alkoxycarbonyl with 1 to 5 carbon atoms in the alkoxy part, halogenoalkyl or halogenoalkoxy or halogenoalkylthio or halogenoalkylsulphonyl with 1 to 5 carbon atoms

and 1 to 5 halogen atoms per halogenoalkyl radical,

$R^1$ and $R^2$ represent identical or different alkyl each with 1 to 5 carbon atoms or

$R^1$ and $R^2$ represent an alkylene bridge with 2 to 8 carbon atoms which, together with the nitrogen on which they are located, form a ring which can be interrupted by further hetero atoms such as oxygen, sulphur, the groups SO or $SO_2$ or by N-alkyl with 1 to 3 carbon atoms in the alkyl part. The rings can optionally be mono- to pentasubstituted by alkyl with 1 to 6 carbon atoms, characterised in that propargylsulphamides of the formula (II)

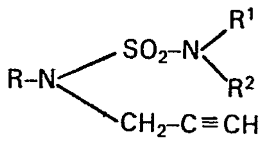

(II)

in which

R, $R^1$ and $R^2$ have the abovementioned meaning, are reacted with iodine in the presence of a base and if appropriate in the presence of a solvent or diluent.

5. Microbicidal agents, characterised in that they contain at least one iodopropargylsulphamide of the formula (I).

6. Use of iodopropargylsulphamides of the formula (I) for combating microorganisms.

7. Method of combating microorganisms, characterised in that iodopropargylsulphamides of the formula (I) are allowed to act on microorganisms and/or their habitat.

8. Process for the preparation of microbicidal agents, characterised in that iodopropargylsulphamides of the formula (I) are mixed with extenders and/or surface-active agents.